Europäisches Patentamt

European Patent Office

Office européen des brevets

(11) Veröffentlichungsnummer: **0 405 287 A1**

(12) **EUROPÄISCHE PATENTANMELDUNG**

(21) Anmeldenummer: 90111496.7

(22) Anmeldetag: 19.06.90

(51) Int. Cl.⁵: **C07C 309/04, C07C 303/02**

(30) Priorität: 28.06.89 DE 3921131

(43) Veröffentlichungstag der Anmeldung:
02.01.91 Patentblatt 91/01

(84) Benannte Vertragsstaaten:
GR

(71) Anmelder: **Henkel Kommanditgesellschaft auf Aktien**
**Henkelstrasse 67**
**D-4000 Düsseldorf 13(DE)**

(72) Erfinder: **Wüst, Willi, Dr.**
**Fasanenring 32**
**D-4030 Ratingen 6(DE)**
Erfinder: **Eskuchen, Rainer, Dr.**
**Benrather Schlossallee 36**
**D-4000 Düsseldorf 13(DE)**
Erfinder: **Lohr, Christoph, Dr.**
**Liboristrasse 45**
**D-4600 Dortmund 1(DE)**

(54) Verfahren zur Herstellung von hellfarbigen niederen Alkansulfonsäuren, insbesondere Methansulfonsäure.

(57) Beschrieben wird ein Verfahren zur Herstellung von hellfarbigen niederen Alkansulfonsäuren, insbesondere Methansulfonsäure durch Umsetzung der entsprechenden Alkylhalogenide mit Alkalisulfit in wäßriger Lösung bei erhöhten Temperaturen und Drucken, nachfolgender Abtrennung des Alkalihalogenids und Gewinnung der freien Alkansulfonsäure. Das Verfahren ist dadurch gekennzeichnet, daß man die Umsetzung als Flüssig-/-Flüssig-Reaktion bei Temperaturen nicht oberhalb etwa 120 °C und derart erhöhten Drucken durchführt, daß auch das niedere Alkylhalogenid bei Reaktionstemperatur als Flüssigphase vorliegt, das gebildete wäßrige Reaktionsgemisch durch Wasserentzug zu einer wäßrigen, auskristallisierte Salzphasen enthaltenden Aufschlämmung mit einem Restwassergehalt von höchstens etwa 50 Gew.-% aufarbeitet, durch Zugabe von HCl die Alkansulfonsäure aus ihrem Alkalisalz freisetzt, die im Reaktionsgemisch jetzt noch vorliegende feste Salzphase abtrennt und die freie Alkansulfonsäure aus der Flüssigphase gewinnt. Zur Gewinnung einer von Chloridionen praktisch freien Alkansulfonsäure wird bevorzugt das durch partiellen Wasserentzug zur wäßrigen Salzaufschlämmung aufkonzentrierte primäre Reaktionsprodukt mit einem mehrfachmolaren Überschuß an HCl - bezogen auf Alkansulfonsäure - versetzt, die jetzt vorliegende Feststoffphase abgetrennt, woraufhin aus der isolierten Flüssigphase der HCl-Überschuß abgezogen und praktisch chloridfreie Alkansulfonsäure zusammen mit dem Restwasser als Sumpfphase der Destillation gewonnen wird.

# VERFAHREN ZUR HERSTELLUNG VON HELLFARBIGEN NIEDEREN ALKANSULFONSÄUREN, INSBESONDERE METHANSULFONSÄURE

Die Erfindung betrifft ein verbessertes Verfahren zur Herstellung von hellfarbigen niederen Alkansulfonsäuren durch Umsetzung der entsprechenden Alkylhalogenide mit Alkalisulfit in wäßriger Lösung bei erhöhten Temperaturen und Drucken und nachfolgender Abtrennung des Alkalihalogenids, Freisetzung der gebildeten Alkansulfonsäure und deren Gewinnung. Die Erfindung will dabei insbesondere Alkansulfonsäuren hoher Reinheit zur Verfügung stellen, die sich beispielsweise für den Einsatz auf dem Gebiet technischer Reinigungsmittel eignen. Hier wird vor allen Dingen die Einhaltung niedriger Maximalwerte an Chloridionen-Verunreinigung in der Alkansulfonsäure gefordert. Großtechnische Mengen derartiger hochreiner Alkansulfonsäuren stehen zu akzeptablen Preisen derzeit nicht zur Verfügung. Ziel der Erfindung ist dementsprechend Zugang zu der hier insbesondere interessierenden Stoffklasse von $C_1$- bis $C_6$-Alkansulfonsäuren, vorzugsweise zu entsprechenden freien Sulfonsäuren mit 1 bis 4 C-Atomen zu schaffen, die gerade auch unter Berücksichtigung des Gestehungspreises in der Praxis einsetzbar sind. Besondere Bedeutung muß hier der Methansulfonsäure zukommen. Die für ihre Herstellung benötigten Einsatzmaterialien stehen als billige Chemikalien in großen Mengen zur Verfügung.

Kurzkettige Alkansulfonsäuren können durch Oxidation der entsprechenden Mercaptane mit Salpetersäure oder Persäuren sowie mit Jod in Gegenwart von Bromidionen in Dimethylsulfoxid hergestellt werden. Diese Verfahren ergeben jedoch nur mäßige Ausbeuten. Zudem sind Mercaptane für großtechnische Verfahren zu teuer.

Kurzkettige Alkansulfonsäuren lassen sich weiterhin aus Alkanen durch Sulfochlorierung mit anschließender Verseifung des Alkansulfonylchlorids oder durch Sulfoxidation herstellen. Die Sulfonierung erfolgt jedoch an der Kohlenwasserstoffkette in statistischer Verteilung und zudem auch gegebenenfalls mehrfach, so daß die selektive Herstellung von 1-Sulfonsäuren nicht möglich ist.

Auch die Umsetzung von Olefinen mit Natriumhydrogensulfit und anschließender Freisetzung der Alkansulfonsäuren ergibt unerwünschte Nebenprodukte.

Aus Bl. Chem. Soc. Japan 32, 850 (1959) ist die Umsetzung von kurzkettigen Alkylhalogeniden mit Natriumsulfit zu den entsprechenden Alkylnatriumsulfonaten nach der sogenannten Strecker-Synthese bekannt. Die Freisetzung der kurzkettigen Alkansulfonsäuren aus den erhaltenen Natriumsalzen ist jedoch nicht beschrieben. Tatsächlich stößt sie auch auf Schwierigkeiten, weil sowohl die kurzkettigen Alkansulfonsäuren als auch ihre Salze ebenso wie die als Reaktionsnebenprodukte entstehenden Natriumsalze in Wasser sehr gut löslich sind. Die einfache Extraktion der gewünschten Alkansulfonsäure aus dem gebildeten Reaktionsgemisch scheidet also aus.

Gegenstand der älteren Anmeldung P 38 12 846.2 (D 7989) "Verfahren zur Herstellung von $C_1$ bis $C_6$-Alkansulfonsäuren" der Anmelderin ist ein Verfahren zur Herstellung geradkettiger oder verzweigter $C_1$ bis $C_6$-Alkansulfonsäuren aus deren Alkalisalzen, wobei dieses Verfahren dadurch gekennzeichnet ist, daß man die Alkalisalze in einer Losung oder Suspension in $C_{1-4}$-Monoalkanolen mit Chlorwasserstoff umsetzt, die ausgefallenen Alkalichloride abtrennt und die $C_{1-6}$-Alkansulfonsäuren aus der Monoalkanolphase isoliert. In den Beispielen dieser älteren Anmeldung wird die Herstellung der jeweiligen Natriumsalze der betroffenen Alkansulfonsäuren nach der Strecker-Synthese durch Umsetzung der entsprechenden Alkylchloride mit Natriumsulfit in wäßriger Lösung bei 150 °C und einer Reaktionszeit von 8 Stunden beschrieben. Das entsprechende Natriumsalz der Methansulfonsäure wird durch eine 12-stündige Umsetzung im Autoklaven bei 100 °C hergestellt. Es stellen sich vergleichsweise beschränkte Reaktionsenddrucke ein.

Die Erfindung geht demgegenüber zunächst einmal von der Aufgabe aus Reaktionsbedingungen aufzuzeigen, nach denen eine substantiell wirtschaftlichere Herstellung der Alkalisalze und insbesondere der Natriumsalze der hier betroffenen niederen Alkansulfonsäuren und insbesondere der Methansulfonsäure möglich wird. Gemäß einer weiteren zusätzlichen Aufgabenstellung der Erfindung soll dann ein einfacher Weg aufgezeigt werden, aus den gebildeten wäßrigen Lösungen der Natriumsalze die freie Alkansulfonsäure abzutrennen, wobei bevorzugt auf die Mitverwendung von Hilfslösungsmitteln im Sinne der niederen Monoalkanole aus der Lehre der genannten älteren Anmeldung verzichtet werden kann.

Gegenstand der Erfindung ist dementsprechend in einer ersten Ausführungsform ein Verfahren zur Herstellung von hellfarbigen niederen Alkansulfonsäuren und insbesondere der Methansulfonsäure durch Umsetzung der entsprechenden Alkylhalogenide in wäßriger Lösung mit Alkalidisulfit bei erhöhten Temperaturen und Drucken mit nachfolgender Abtrennung des Alkalihalogenids und Gewinnung der freien Alkansulfonsäure. Das neue Verfahren ist dadurch gekennzeichnet, daß man die Umsetzung als Flüssig-/-Flüssig-Reaktion bei Temperaturen von höchstens etwa 120 °C und derart erhöhten Drucken durchführt, daß auch das eingesetzte niedere Alkylhalogenid bei Reaktionstemperatur als Flüssigphase vorliegt, daß man weiterhin das gebildete wäßrige Reaktionsgemisch durch partiellen Wasserentzug zu einer wäßrigen, auskristallisierte Salzphasen enthaltenden Aufschlämmung mit einem Restwassergehalt von höchstens etwa 50 Gew.-% aufarbeitet, dann durch Zugabe von Chlorwasserstoff die Alkansulfonsäure aus ihrem Alkalisalz freisetzt, die im Reaktionsgemisch jetzt noch vorliegende feste Salzphase abtrennt und die freie Alkansulfonsäure aus der Flüssigphase gewinnt.

Im nachfolgenden wird die Lehre der Erfindung anhand der Herstellung von Methansulfonsäure unter Einsatz von Methylhalogenid insbesondere Methylchlorid geschildert. Sie ist aber sinngemäß anwendbar auch auf die Herstellung anderer niederer Alkansulfonsäuren der geschilderten Art, insbesondere des Bereichs mit bis zu 6, vorzugsweise bis zu 4 C-Atomen.

Die Umsetzung des Methylchlorids mit der Sulfitionen liefernden Reaktionskomponente erfolgt in wäßriger Lösung und dabei unter solchen Bedingungen von Druck und Temperatur, daß die Reaktion als Flüssig-/Flüssig-Umsetzung abläuft. Zur Unterbindung der Bildung unerwünschter Nebenprodukte werden Reaktionstemperaturen unterhalb 100 °C und vorzugsweise Temperaturen von höchstens 90 °C eingesetzt. Besonders geeignet sind maximale Reaktionstemperaturen bis etwa 80 °C. Da andererseits die Reaktionsgeschwindigkeit bei Temperaturen unterhalb 70 °C sehr stark absinkt, hat sich besonders der Bereich von etwa 70 bis 80 °C als besonders geeignet erwiesen.

Die eingesetzten Reaktionsdrucke liegen oberhalb 10 bar und vorzugsweise bei wenigstens 15 bar. Besonders geeignet ist der Druckbereich von 15 bis 30 bar, für die Herstellung der Methansulfonsäure insbesondere der Druckbereich von 18 bis 20 bar.

Die Flüssig-/Flüssig-Reaktion kann unter diesen Arbeitsbedingungen beispielsweise in einem als Rührkessel ausgebildeten Druckreaktor durchgeführt werden. Es hat sich gezeigt, daß die vergleichsweise hohen Reaktionsdrucke zu einer starken Beschleunigung der Umsetzung führen, so daß im angegebenen Druckbereich bei Reaktionszeiten von höchstens etwa einer Stunde, bevorzugt bei Reaktionszeiten bis zu etwa einer halben Stunde gearbeitet werden kann und gleichwohl die angestrebten Umsetzungsgrade erreicht sind. In einer bevorzugten Ausführungsform zur Herstellung von Methansulfonsäure reichen unter den angegebenen bevorzugten Temperatur- und Druckbedingungen Reaktionszeiten von etwa 2 bis 20 Minuten aus, um die angestrebte Umsetzung zu bewerkstelligen.

Als Sulfit-liefernder Reaktant kann Natriumsulfit in wäßriger Lösung eingesetzt werden. In einer bevorzugten Ausführungsform der Erfindung wird allerdings das preisgünstigere Alkalidisulfit, insbesondere also das Natriumdisulfit $Na_2S_2O_5$ verwendet, das zusammen mit stöchiometrischen Mengen an Alkalihydroxid, insbesondere Natriumhydroxid zur intermediären Bildung von Natriumsulfit führt. In dieser Ausführungsform können die Kosten des insgesamt benötigten Einsatzmaterials nochmals substantiell gesenkt werden. Die Umsetzung des Natriumdisulfits mit Natriumhydroxid in wäßriger Lösung wird zweckmäßigerweise vor der Einspeisung des Methylchlorids in die wäßrige Reaktionsphase vorgenommen.

Nach Abschluß der Umsetzung zwischen dem Alkylhalogenid, insbesondere also dem Methylchlorid und der wäßrigen Natriumsulfitlösung wird die Aufarbeitung des gebildeten Reaktionsgemisches vorgenommen. Hierbei kann im Sinne der Lehre der genannten älteren Anmeldung P 38 12 846.2 (D 7989) vorgegangen werden. In einer erfindungsgemäß besonders wichtigen Ausführungsform werden allerdings in dieser Aufarbeitungsstufe Verfahrenselemente eingesetzt, wie sie in der parallelen Schutzrechtsanmeldung P ... (D 8743 "Verfahren zur Herstellung von niederen Alkansulfonsäuren aus ihren Alkalisalzen") geschildert sind. Die Lehre der Erfindung vereinigt in dieser Ausführungsform die Verfahrensmerkmale beider Schutzrechtsanmeldungen. Zum Zwecke der vollständigen Erfindungsoffenbarung werden im nachfolgenden die wesentlichen Maßnahmen der hier folgenden weiteren Aufarbeitung nochmals geschildert:

In dieser bevorzugten Ausführungsform wird derart gearbeitet, daß man zur Gewinnung einer an Chloridionen praktisch freien Alkansulfonsäure deren Alkalisalze, gewünschtenfalls in Abmischung mit Alkalichlorid, in Form einer aufkonzentrierten wäßrigen Salzaufschlämmung mit einem mehrfachmolaren Überschuß an Chlorwasserstoff - bezogen auf Alkansulfonsäure -versetzt, die jetzt vorliegende Feststoffphase abtrennt, aus der isolierten Flüssigphase den Chlorwasserstoff-Überschuß gewünschtenfalls zusammen mit einem Anteil des Wassers destillativ abzieht und die praktisch chloridfreie Alkansulfonsäure zusammen mit dem Restwasser als Sumpfphase der Destillation gewinnt. Bevorzugt wird das primär angefallene Reaktionsprodukt vor der HCl-Zugabe soweit aufkonzentriert, daß eine gerade noch fließ und pumpfähige Salzaufschlämmung vorliegt.

Kern des erfindungsgemäßen Handelns ist hier die überraschende Feststellung, daß durch Einsatz eines beträchtlichen Überschusses an HCl bei der Umwandlung des in wäßriger Lösung bzw. Aufschlämmung vorliegenden Alkalisalzes der Alkansulfonsäuren unter Freisetzung der freien Säure und gleichzeitiger Bildung des Alkalichlorids die Löslichkeit des Alkalichlorids in der aufkonzentrierten wäßrigen Phase so stark abgesenkt werden kann, daß die praktisch quantitative Entfernung des Alkalichlorids durch einfache Phasentrennung gelingt. In der auf diese Weise zu ge winnenden wäßrigen Flüssigphase liegt die freie Alkansulfonsäure zusammen mit dem eingesetzten HCl-Überschuß vor. Auf die im nachfolgenden noch zu schildernde Weise gelingt unter schonenden Bedingungen die Abtrennung des Chlorwasserstoffs, dessen Restmengen als wäßriges Azeotrop auf destillativem Wege abgezogen werden können, so daß schließlich eine hellfarbige und einstellbare Restwassermengen enthaltende freie Alkansulfonsäure gewonnen werden kann, die praktisch chloridionenfrei ist.

Diese Ausführungsform der Aufarbeitung eignet sich insbesondere für die Herstellung von C1- bis C4-Alkansulfonsäuren. Insbesondere die Methansulfonsäure läßt sich aus ihren Alkalisalzen durch das neue Verfahren in einfacher Weise gewinnen. Die bevorzugten Alkalisalze sind im Rahmen der erfindungsgemäß geschilderten Umsetzung die Natriumsalze.

Die entscheidende Arbeitshilfe, durch die das Arbeiten in wäßrigem Medium beim hier geschilderten Reaktionstyp möglich wird, ist der Einsatz des Chlorwasserstoffs im Überschuß bei der Freisetzung der Alkansulfonsäure, und zwar in mehrfach molarem Überschuß. Vorzugsweise wird Chlorwasserstoff wenigstens in etwa 3-molarer Menge bezogen auf vorliegendes Alkalisalz der Alkansulfonsäure verwendet. Dieser HCl-Überschuß ist für die Gesamtbilanz des Verfahrens unproblematisch, weil die zur Salzbildung nicht benötigten Anteile des Chlorwasserstoffs im Kreislauf wieder in die nächste Verfahrensstufe zurückgeführt werden können. Üblicherweise wird mit HCl-Mengen von höchstens etwa 5 Mol - bezogen auf das Alkalisalz der Alkansulfonsäure -gearbeitet. Als besonders geeignet hat sich ein Bereich von etwa 3,2 bis 4 Mol HCl erwiesen.

Nach der Behandlung des als wäßrige Salzaufschlämmung eingesetzten Alkalisalzes der Alkansulfonsäure mit dem Chlorwasserstoff, der in freier Form und/oder als wäßrige Lösung - bei spielsweise als rauchende Salzsäure - eingesetzt werden kann, bildet sich eine Aufschlämmung von Alkalichlorid insbesondere Natriumchlorid in einer Flüssigphase aus, die die freie Alkansulfonsäure zusammen mit HCl gelöst enthält. Die Löslichkeit des Natriumchlorids in dieser Flüssigkeit ist durch HCl-Überschuß verschwindend gering. Das als Feststoff vorliegende Natriumchlorid wird in geeigneter Weise beispielsweise durch Filtration oder Zentrifugieren von der Flüssigphase abgetrennt. Diese Trennung kann bei Normaltemperatur oder bestenfalls mäßig erhöhten Temperaturen, also beispielsweise im Temperaturbereich bis etwa 60 °C durchgeführt werden. Häufig liegen zum Zeitpunkt der Phasentrennung Aufschlämmungen im Temperaturbereich von etwa 40 bis 60 °C vor, die sich bei diesen Temperaturen für die Trennstufe eignen.

Die nachfolgende Auftrennung der gewonnenen Flüssigphase kann auf destillativem Wege, und zwar insbesondere in mehrstufiger Form erfolgen. In der einfachsten Form wird hier eine zweistufige destillative Trennung derart vorgenommen, daß zunächst der Chlorwasserstoff abgetrieben und in das Hauptverfahren zurückgeführt wird. In der Endstufe dieser Auftrennung der Flüssigphase kann dann ein HCl/Wasser-Gemisch abgetrennt werden. Bevorzugt wird hier im schwachen Vakuum gearbeitet, so daß Temperaturen unter 100 °C eingehalten werden können, wobei insbesondere Temperaturen der Flüssigphase im Bereich von etwa 80 bis 90 °C geeignet sein können. Auch dieses in zweiter Verfahrensstufe abgetrennte HCl/Wasser-Azeotrop kann in die Hauptreaktion zurückgeführt werden, es ist dann jeweils nur der im Kreislauf geführte Wasserbetrag mit zu berücksichtigen.

Nach Abtrennung des Chlorwasserstoffs und begrenzter Wassermengen aus der Flüssigphase fällt als Sumpfphase die freie Alkansulfonsäure als hellfarbiges chloridfreies Reaktionsprodukt an. In einer bevorzugten Ausführungsform der Erfindung wird dabei die Wasserbilanz des Gesamtverfahrens so geregelt, daß die hier anfallende freie Alkansulfonsäure auch ihrerseits noch einen geringen Restwassergehalt von wenigstens etwa 1 bis maximal etwa 30 Gew.-% und bevorzugt von etwa 10 bis 20 Gew.-% aufweist. Hierzu ist es lediglich notwendig, den Wassergehalt der für die Freisetzungsstufe der Alkansulfonsäure eingesetzten wäßrigen Salzaufschlämmung so zu wählen, daß trotz Abziehens eines Anteils der wäßrigen Phase mit den restlichen überschüssigen HCl-Mengen bei der vollständigen Entfernung der HCl auf destillativem Wege der angestrebte Wassergehalt in Mischung mit der freigesetzten Alkansulfonsäure zurückbleibt.

Die so gewonnenen hochkonzentrierten Alkansulfonsäuren können - gewünschtenfalls nach Verdünnung mit weiterem Wasser - dem jeweiligen Verwendungszweck zugeführt werden. Der Chloridgehalt der Alkansulfonsäuren liegt im akzeptablen Spurenbereich. So ist es beispielsweise ohne weiteres möglich, auf dem geschilderten Weg Methansulfonsäure aus ihrem Natriumsalz mit Chloridgehalten deutlich unter 500 ppm zu gewinnen.

Die zuvor geschilderte destillative Abtrennung der überschüssigen Salzsäure, insbesondere in der Form eines wäßrigen Azeotrops kann beispielsweise in Fallfilmverdampfern erfolgen, die materialmäßig so ausgelegt sind, daß sie durch das an sich aggressive Medium nicht geschädigt werden. Materialien bzw. Vorrichtungen dieser Art sind im Stand der Technik bekannt. Zu nennen sind hier beispielsweise entsprechende Vorrichtungen auf Graphitbasis.

## Beispiele

### Versuchsaufbau

Die Versuche wurden in einem 2.5l Autoklaven ($P_{max.}$ 30 bar), der mit einem Ankerrührer ausgestattet war, durchgeführt. Die Zuführung des Methylchlorids erfolgte über eine angeflanschte Gasbombe. Zur Heizung und Kühlung war der doppelmantige Autoklav an einen Thermostaten angeschlossen.

### Versuchsdurchführung

Im Reaktor vorgelegt wurde eine aus festem $Na_2S_2O_5$ und $H_2O$ hergestellte Natriumhydrogensulfitlösung, die im Reaktor mit der stöchiometrischen Menge 50 % NaOH zur Sulfitlösung umgesetzt wurde. In die auf 60 °C aufgeheizte Reaktionslösung wurde nun aus einer Gasbombe das Methylchlorid sehr schnell in den Reaktor hineingedrückt. Um eine schnelle Zudosierung des Flüssiggases zu ermöglichen, war die Bombe zuvor mit $N_2$ auf einen Innendruck von ca. 30 bar gebracht worden.

### Beispiel 1 (Primärreaktion)

1500 g einer aus $Na_2S_2O_5$ und $H_2O$ hergestellten 20 Gew.-% Hydrogensulfit wurden im Reaktor vorgelegt und mit 230 g NaOH (50 %) zu einer 21 % Natriumsulfitlösung umgesetzt. Nach Aufheizen auf 60 °C wurden 146 g Methylchlorid aus einer Gasbombe nach dem oben beschriebenen Verfahren innerhalb kurzer Zeit in den Reaktor eingeleitet. Die Reaktionslösung erhitzte sich dabei auf 85 °C, der Druck stieg kurzzeitig auf 24 bar. Nach ca. 90 Sekunden war die Reaktion beendet.
Die Zusammensetzung der Lösung in Gew.-%:
$CH_3SO_3^-$: 14,5 %
$SO_3$: weniger als 0,1 %
$Cl^-$: 5,4 %
Methanol: weniger als 200 ppm
Dimethyläther: weniger als 50 ppm

### Beispiel 2 (Primärreaktion)

1500 g einer aus $Na_2S_2O_5$ und $H_2O$ hergestellten 28,5 Gew.-% Hydrogensulfit wurden im Reaktor vorgelegt und mit 360 g NaOH (50 %) zu einer 30,5 % Natriumsulfitsuspension umgesetzt. Nach Aufheizen auf 60 °C erfolgte das rasche Einleiten von 230 g Methylchlorid aus einer Gasbombe nach dem oben beschriebenen Verfahren. Die Reaktionslösung erwärmte sich zunächst rasch auf 80 °C, bis zur vollständigen Umsetzung der Edukte mußte für 20 min. eine Temperatur von 75 °C mit Hilfe des Thermostaten gehalten werden. Nach Reaktionsende besaß die jetzt klare Salzlösung folgende Zusammensetzung:
$CH_3SO_3^-$: 20 %
$SO_3$: weniger als 0,1 %
$Cl^-$: 7,6 %
Methanol: weniger als 200 ppm
Dimethyläther: weniger als 50 ppm

### Aufarbeitung zur freien Säure

1000 g der gemäß Beispielen 1 und 2 hergestellten Lösungen wurden im Wasserstrahlvakuum am Rotationsverdampfer bis auf eine geringe Restfeuchte (weniger als 5 %), eingeengt. 300 g des so erhaltenen Salzes wurden in 450 g rauchender Salzsäure (37 %) aufgenommen. Nach dem Abfiltrieren des ausgefallenen NaCl wurde die Salzsäure-Methansulfonsäure-Mischung am Rotationsverdampfer bei 110 °C im Wasserstrahlvakuum auf eine Konzentration von 80 Gew.-% Methansulfonsäure und 20 % Wasser eingeengt. Die Chloridkonzentration betrug weniger als 200 ppm $Cl^-$, die Ausbeute 98 % bezogen auf das eingesetzte Sulfonatsalz.

## Ansprüche

1. Verfahren zur Herstellung von hellfarbigen niederen Alkansulfonsäuren, insbesondere Methansulfonsäure durch Umsetzung der entsprechenden Alkylhalogenide mit Alkalisulfit in wäßriger Lösung bei erhöhten Temperaturen und Drucken, nachfolgender Abtrennung des Alkalihalogenids und Gewinnung der freien Alkansulfonsäure, dadurch gekennzeichnet, daß man die Umsetzung als Flüssig-/Flüssig-Reaktion bei Temperaturen nicht oberhalb etwa 120 °C und derart erhöhten Drucken durchführt, daß auch das niedere Alkylhalogenid bei Reaktionstemperatur als Flüssigphase vorliegt, das gebildete wäßrige Reaktionsgemisch durch Wasserentzug zu einer wäßrigen, auskristallisierte Salzphasen enthaltenden Aufschlämmung mit einem Restwassergehalt von höchstens etwa 50 Gew.-% aufarbeitet, durch Zugabe von HCl die Alkansulfonsäure aus ihrem Alkalisalz freisetzt, die im Reaktionsgemisch jetzt noch vorliegende feste Salzphase abtrennt und die freie Alkansulfonsäure aus der Flüssigphase gewinnt.

2. Verfahren nach Anspruch 1, dadurch gekennzeichnet, daß man mit Alkylchloriden mit insbesondere 1 bis 4 C-Atomen im Alkylrest, vorzugsweise mit Methylchlorid als Einsatzmaterial arbeitet.

3. Verfahren nach Ansprüchen 1 und 2, dadurch gekennzeichnet, daß man eine wäßrige Lösung von Alkalidisulfit - insbesondere $Na_2S_2O_5$ - und zur Bildung von Alkalisulfit stöchiometrischen Mengen an Alkalihydroxid - insbesondere NaOH - zur Umsetzung mit dem Alkylhalogenid einsetzt.

4. Verfahren nach Ansprüchen 1 bis 3, dadurch gekennzeichnet, daß man in der ersten Verfahrensstufe bei Drucken von wenigstens 10 bar, im Falle der Umsetzung des Methylchlorids vorzugsweise von wenigstens 15 bar und bei Temperaturen nicht oberhalb 100 °C arbeitet.

5. Verfahren nach Ansprüchen 1 bis 4, dadurch gekennzeichnet, daß man die Umsetzung von Methylchlorid im Druckbereich von 15 bis 30 bar, im Temperaturbereich von etwa 70 bis 80 °C und bei Reaktionszeiten unterhalb 60 Minuten, insbesondere im Bereich von etwa 2 bis 20 Minuten vornimmt.

6. Verfahren nach Ansprüchen 1 bis 5, dadurch gekennzeichnet, daß man die partielle Entwässerung des primären Reaktionsproduktes bei Temperaturen unterhalb 100 °C, vorzugsweise im leichten Vakuum durchführt.

7. Verfahren nach Ansprüchen 1 bis 6, dadurch gekennzeichnet, daß man zur Gewinnung einer an Chloridionen praktisch freien Alkansulfonsäure das durch partiellen Wasserentzug zur wäßrigen Salzaufschlämmung aufkonzentrierte primäre Reaktionsprodukt mit einem mehrfach-molaren Überschuß an HCl - bezogen auf Alkansulfonsäure - versetzt, die jetzt vorliegende Feststoffphase abtrennt, aus der isolierten Flüssigphase den HCl-Überschuß gewünschtenfalls zusammen mit einem Teil des verbliebenen Wassers destillativ abzieht und die praktisch chloridfreie Alkansulfonsäure zusammen mit dem Restwasser als Sumpfphase der Destillation gewinnt.

8. Verfahren nach Ansprüchen 1 bis 7, dadurch gekennzeichnet, daß man bei der Freisetzung der Alkansulfonsäure im primär angefallenen Reaktionsgemisch die HCl in wenigstens etwa 3-molarer Menge, vorzugsweise in Mengen von etwa 3,2 bis 4 Mol HCl - jeweils bezogen auf Alkalisalz der Alkansulfonsäure - einsetzt, wobei der in der destillativen Trennstufe zurückgewonnene HCl-Anteil bevorzugt im Kreislauf in das Aufarbeitungsverfahren zurückgeführt wird.

9. Verfahren nach Ansprüchen 1 bis 8, dadurch gekennzeichnet, daß die destillative HCl-Abtrennung mehrstufig, insbesondere zweistufig derart erfolgt, daß zunächst HCl und dann ein HCl/$H_2O$-Gemisch abgetrennt werden, wobei die Destillation bevorzugt wenigstens anteilsweise im Vakuum bei Temperaturen unterhalb 100 °C durchgeführt wird.

10. Verfahren nach Ansprüchen 1 bis 9, dadurch gekennzeichnet, daß in der zweiten Stufe der destillativen Abtrennung ein HCl/Wasser-Azeotrop zurückgewonnen und dabei bevorzugt im schwachen Vakuum im Temperaturbereich von etwa 80 bis 90 °C gearbeitet wird.

11. Verfahren nach Ansprüchen 1 bis 10, dadurch gekennzeichnet, daß bei der Aufkonzentration des wäßrigen primären Reaktionsproduktes solche Restwassergehalte in der entstehenden Salzaufschlämmung eingestellt werden, daß nach der destillativen Abtrennung des HCl/Wasser-Azeotrops eine freie Alkansulfonsäure mit einem Restwassergehalt von wenigstens etwa 1 bis 30 Gew.-%, bevorzugt von etwa 10 bis 20 Gew.-% als Sumpfphase anfällt.

| | Europäisches Patentamt | **EUROPÄISCHER RECHERCHENBERICHT** | Nummer der Anmeldung EP 90 11 1496 |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| P,A D | DE-A-3 812 846 (HENKEL) --- | | C 07 C 309/04 C 07 C 303/02 |
| A | US-A-3 480 636 (B. LOEV) --- | | |
| A | US-A-3 453 320 (M.O. ROBINSON) --- | | |
| A | US-A-2 278 064 (M. DE SIMO et al.) ----- | | |

| | RECHERCHIERTE SACHGEBIETE (Int. Cl.5) |
|---|---|
| | C 07 C 303/00 C 07 C 309/00 |

Der vorliegende Recherchenbericht wurde für alle Patentansprüche erstellt

| Recherchenort | Abschlußdatum der Recherche | Prüfer |
|---|---|---|
| DEN HAAG | 20-09-1990 | VAN GEYT J.J.A. |

EPO FORM 1503 03.82 (P0400)